# EUROPÄISCHE PATENTSCHRIFT

(11) **EP 0 692 476 B1**
(45) Veröffentlichungstag und Bekanntmachung des Hinweises auf die Patenterteilung: **29.12.1999**
(21) Anmeldenummer: 95110050.2
(22) Anmeldetag: 28.06.1995
(51) Int. Cl.: C07D 233/60, C07D 235/20, C07D 249/04, C07D 249/08, C07D 205/06, C07D 521/00, C07D 257/04

(54) **Verfahren zur Herstellung von N,N'-Carbonyldiazolen, insbesondere N,N'-Carbonyldiimidazol**
Process for the preparation of N,N'-carbonyldiazoles, specifically N,N-carbonyl-diimidazole
Procédé pour la préparation de N,N'-carbonyldiazoles, spécialement de N,N'-carbonyldiimidazole

(30) Priorität: 11.07.1994 DE 4424400
(43) Veröffentlichungstag der Anmeldung: 17.01.1996
(73) Patentinhaber: BAYER AG, 51368 Leverkusen (DE)
(72) Erfinder: Antons, Stefan, Dr., D-51373 Leverkusen (DE); Fiege, Helmut, Dr., D-51373 Leverkusen (DE)

(56) Entgegenhaltungen:
- DE-B- 1 033 210
- US-A- 4 942 248
- CHEMISCHE BERICHTE, Bd. 96, Nr. 12, 1963, Seite 3374 XP002033933 STAAB A. HEINZ: "Notiz zur Darstellung von 1.1'-Carbonyl-di-imidazol"

## Beschreibung

Die vorliegende Erfindung betrifft ein verbessertes Verfahren zur Herstellung von N,N'-Carbonyldiazolen durch Umsetzung von Azolen mit Phosgen.

Es ist bereits grundsätzlich bekannt, daß man N,N'-Carbonyldiimidazole, -triazole und -tetrazole erhalten kann, wenn man Imidazole, Triazole oder Tetrazole mit Phosgen umsetzt (siehe DE-AS 1 033 210). Dabei werden Tetrahydrofuran, andere Ether und Kohlenwasserstoffe als Lösungsmittel vorgeschlagen, insbesondere wasserfreies Tetrahydrofuran. Die Umsetzung erfolgt offensichtlich bei Raumtemperatur. Nachteilig ist die Schwierigkeit, die für dieses Verfahren benötigten, auch von letzten Spuren Wasser befreiten Lösungsmittel herzustellen, da dies den Umgang mit metallischen Natrium, Natriumhydrid oder Calciumhydrid erfordert. Das bedeutet für das Arbeiten im technischen Maßstab die Anwendung außerordentlicher Sicherheitsmaßnahmen und kostenintensiver Trocknungsmethoden. Zusätzlich besteht bei Tetrahydrofuran und anderen Ethern stets die Gefahr der Bildung von explosionsfähigen Peroxiden. Außerdem ist das Verfahren nur schwierig reproduzierbar.

Später wird von denselben Autoren ein besser reproduzierbares Verfahren angegeben, bei dem in wasserfreies Benzol zunächst Phosgen eingeleitet und dann unter Kühlung das Imidazol, gelöst in wasserfreiem Tetrahydrofuran zugefügt wird (siehe Chem. Ber 96, 3374 (1963) und Org. Synth. Coll. Vol. IV, 201 - 204 (1968)). Auch hier besteht die Notwendigkeit auf aufwendige Weise wasserfrei zu machende Lösungsmittel einzusetzen, wobei zusätzlich die Problematik der Tetrahydrofuranperoxide bestehen bleibt. N,N'-Carbonyldiimidazol kann so in Ausbeuten von 80-94 % und in einer Reinheit von 98 % erhalten werden. Es weist dann Schmelzpunkte im Bereich 114-118°C auf.

Es wurde nun ein Verfahren zur Herstellung von N,N'-Carbonyldiazolen der Formel (I) gefunden 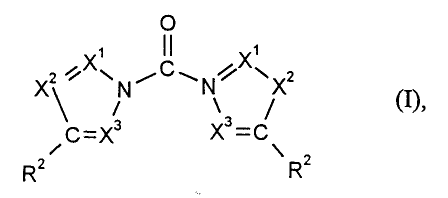 in der
- X¹, X² und X³: unabhängig voneinander jeweils für CR¹ oder Stickstoff stehen, wobei R¹ Wasserstoff oder C₁-C₆-Alkyl bedeutet und
- R²: für Wasserstoff steht oder
- X¹ und X³: für CR¹ stehen,
wobei das an X¹ befindliche R¹ Wasserstoff oder C₁-C₆-Alkyl bedeutet und das an X³ befindliche R¹ gemeinsam mit R² eine -CH=CH-CH=CH-Brücke bilden,
bei dem Azole der Formel (II) 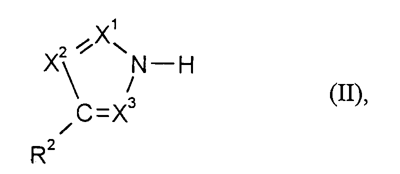 in der die verwendeten Symbole die bei Formel (I) angegebene Bedeutung haben, mit Phosgen in einem Lösungsmittel umsetzt, das dadurch gekennzeichnet ist, daß man die Umsetzung bei 50 bis 120°C und in einem aromatischen Lösungsmittel durchführt, das durch Andestillieren entwässert worden ist.

Bevorzugt wird in das erfindungsgemäße Verfahren nur ein Azol der Formel (II) eingesetzt, und so ein N,N'-Carbonyldiazol der Formel (I) erhalten, bei dem die beiden Azolringe identisch sind.

Weiterhin ist es bevorzugt, daß in den Formeln (I) und (II) ein oder zwei der Molekülteile X¹, X² und X³ für Stickstoff stehen. Außerdem ist bevorzugt, daß X¹ für CH, X² für Stickstoff und X³ für C-R¹, wobei R¹ und R² gemeinsam eine -CH=CH-CH=CH-Brücke bilden.

Besonders bevorzugt setzt man in das erfindungsgemäße Verfahren Imidazol, Benzimidazol, Pyrazol oder 1,2,4-Triazol als Azol der Formel (II) ein. Ganz besonders bevorzugt ist Imidazol.

Phosgen kann in üblicher technischer Qualität eingesetzt werden. Es ist vorteilhaft, pro Mol Azol der Formel (II) 0,2 bis 0,3 Mol Phosgen einzusetzen. Besonders bevorzugt setzt man 0,25 Mol Phosgen pro Mol Azol der Formel (II) ein.

Als aromatische Lösungsmittel kommen beispielsweise Benzol, Toluol, Xylole, Monochlorbenzol, Dichlorbenzole, Trichlorbenzole und Gemische dieser Lösungsmittel in Frage. Bevorzugt sind Toluol und Monochlorbenzol. Überraschenderweise hat sich gezeigt, daß eine aufwendige Trocknung der Lösungsmittel nicht notwendig ist. Die Ausbeute der erfindungsgemäß erhaltenen N,N'-Carbonyldiazolen ist gegenüber dem Stand der Technik gleich oder besser, deren Reinheit praktisch gleich, obwohl erfindungsgemäß die Lösungsmittel nur durch Andestillieren entwässert werden.

Pro Mol eingesetzter Verbindung der Formel (II) kann man beipielsweise 200 bis 2 000 g Lösungsmittel einsetzen.

Das Andestillieren zur Entwässerung der Lösungsmittel kann z.B. so geschehen, daß man das jeweilige Lösungsmittel vor dem Einsatz bei normalem oder erniedrigtem Druck so lange auf Siedetemperatur erhitzt, bis kein Austragen von Wasser mehr beobachtet wird. Im allgemeinen wird dabei zwischen 0,1 und 5, vorzugsweise zwischen 0,5 und 2 Gew.-% des Lösungsmittels abdestilliert. Das danach hinterbleibende Lösungsmittel ist dann für das erfindungsgemäße Verfahren ausreichend entwässert.

Das erfindungsgemäße Verfahren wird bei 50 bis 120°C durchgeführt. Vorzugsweise arbeitet man im Bereich 60 bis 100°C.

Es ist im allgemeinen vorteilhaft, nach dem Zusammengeben der Reaktanden das Reaktionsgemisch noch einige Zeit z.B. 30 Minuten bis 5 Stunden, bei erhöhter Temperatur nachzurühren.

Das erhaltene Reaktionsgemisch kann man z.B. so aufarbeiten, daß man das gebildete Azolhydrochlorid bei normaler oder erhöhter Temperatur durch Filtration abtrennt und aus der Mutterlauge durch Eindampfen, vorzugsweise bei vermindertem Druck, das hergestellte N,N'-Carbonyldiazol in fester Form erhält.

Man kann so N,N'-Carbonyldiazole in guten Ausbeuten und Reinheiten erhalten. Im Falle der Herstellung von N,N'-Carbonyldiimidazol liegen die Ausbeuten meist bei über 94 % und die Reinheiten bei über 95 %.

Bei einer bevorzugten technischen Ausführungsform der vorliegenden Erfindung wird wie folgt gearbeitet:

Das einzusetzende Lösungsmittel wird vorgelegt, bei Normaldruck bis zum Sieden erhitzt und ca. 1 bis 2 Gew.-% des Lösungsmittels abdestilliert. Danach wird die Verbindung der Formel (II) in das hinterbliebene Lösungsmittel gegeben, auf einige °C unterhalb der gewünschten Reaktionstemperatur erhitzt und unter Rühren in Lösung gebracht. Danach werden pro Mol der Verbindung der Formel (II) ca. 0,25 Mol Phosgen eingeleitet, wobei die Temperatur etwas ansteigt. Nach beendeter Phosgenzugabe wird noch ca. 1 Stunde nachgerührt, dann gegebenenfalls überschüssiges Phosgen mit Stickstoff oder einem anderen inerten Gas ausgeblasen. Das gebildete Hydrochlorid wird nunmehr bei 50 bis 120°C durch Filtration abgetrennt, der Filterkuchen mit frischem, entwässertem Lösungsmittel bei 50 bis 120°C nachgewaschen, das Filtrat und die Waschflüssigkeit vereinigt und dieses Gemisch bei vermindertem Druck vom Lösungsmittel befreit.

Es ist ausgesprochen überraschend, daß es mit dem erfindungsgemäßen Verfahren gelingt mit günstig zur Verfügung stehenden Lösungsmitteln und ohne aufwendige Entwässerungsverfahren für die Lösungsmittel bei erhöhter Temperatur N,N'-Carbonyldiazole in so guten Ausbeuten und Reinheiten zu erhalten.

### Beispiele

### Beispiel 1

In 1 000 g entwässertes Monochlorbenzol, das durch Andestillieren von 1 010 g Monochlorbenzol bei 140°C erhalten worden war, wurden bei Raumtemperatur 136 g Imidazol eingetragen und auf 60°C erhitzt. Dabei bildete sich eine klare Lösung. Dann wurden bei 60 bis 70°C im Verlaufe von 35 Minuten 50 g Phosgen unter Rühren eingeleitet. Nach Beendigung der Phosgenzugabe wurde noch 1 Stunde bei 65°C nachgerührt, danach restliches Phosgen mit Stickstoff ausgeblasen. Das ausgefallene Imidazolhydrochlorid wurde bei 100°C abfiltriert und mit 100 ml entwässertem Chlorbenzol bei 100°C gewaschen. Die Mutterlauge und die Waschflüssigkeit wurden vereinigt und das darin enthaltene Monochlorbenzol bei 60°C und 200 mbar eingedampft. So wurden 80,2 g N,N'-Carbonyldiimidazol mit einem Schmelzpunkt von 118°C und einer gaschromatographisch bestimmten Reinheit von 98,5 % erhalten.

### Beispiele 2 bis 4

Es wurde verfahren wie in Beispiel 1, jedoch wurden die Reaktionstemperaturen variiert. Die Ergebnisse sind aus der nachfolgenden Tabelle 1 ersichtlich.

### Beispiele 5 bis 7 (zum Vergleich)

Es wurde verfahren wie in Beispiel 1, jedoch wurde bei Temperaturen außerhalb des erfindungsgemäß einzuhaltenden Bereichs und mit verschiedenen Lösungsmitteln gearbeitet. Die Ergebnisse sind aus der nachfolgenden Tabelle 1 ersichtlich.

**Tabelle 1**

| Beispiel Nr. | Lösungsmittel | Reaktions-temperatur (°C) | Ausbeute, isoliert (% d.Th.) | Schmelzpunkt des Produkts (°C) | Reinheit des Produkts nach GC (Gew.-%) |
|---|---|---|---|---|---|
| 2 | Chlorbenzol | 60-80°C | >95 | 120 | 98 |
| 3 | Chlorbenzol | 60 | >95 | 120 | 98 |
| 4 | Chlorbenzol | 100 | 94 | 114-117 | 95 |
| 5*⁾ | Chlorbenzol | 10-15 | 51 | 100-104 | <90 |
| 6*⁾ | Toluol | 10-15 | <10 | 80 | <90 |
| 7*⁾ | Methylenchlorid | 42 | 62 | 117-119 | 96 |

| | | | | | |
|---|---|---|---|---|---|
| *⁾Vergleichsbeispiele | | | | | |

### Beispiel 8

Es wurde verfahren wie in Beispiel 1, jedoch wurde o-Dichlorbenzol als Lösungsmittel eingesetzt. N,N'-Carbonyldiimidazol wurde in einer Ausbeute von über 95 % der Theorie und mit einer Reinheit von 98 % (Schmelzpunkt: 119 bis 120°C) erhalten.

### Beispiele 9 bis 11

Es wurde verfahren wie in Beispiel 1, jedoch wurden anstelle von Imidazol 1,2,4-Triazol (Beispiel 9), Pyrazol (Beispiel 10) und Benzimidazol (Beispiel 11) eingesetzt. Weitere Einzelheiten sind aus der nachfolgenden Tabelle 2 ersichtlich.

**Tabelle 2**

| Beispiel Nr. | Reaktionsprodukt | Ausbeute, isoliert (% d. Th.) | Schmelzpunkt des Produktes (°C) |
|---|---|---|---|
| 9 | N,N'-Carbonyldi(1,2,4-triazol) | 90 | 149-150 |
| 10 | N,N'-Carbonyldipyrazol | >95 | 55-56 |
| 11 | N,N'-Carbonyldibenzimidazol | 62 | 181 |

## Patentansprüche

1. Verfahren zur Herstellung von N,N'-Carbonyldiazolen der Formel (I) 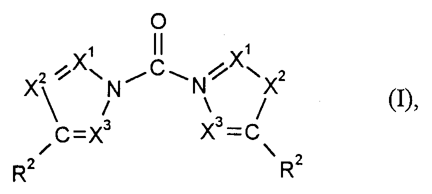 in der
X¹, X² und X³ unabhängig voneinander jeweils für CR¹ oder Stickstoff stehen, wobei R¹ Wasserstoff oder C₁-C₆-Alkyl bedeutet und
R² für Wasserstoff steht oder
X¹ und X³ für CR¹ stehen,
wobei das an X¹ befindliche R¹ Wasserstoff oder C₁-C₆-Alkyl bedeutet und das an X³ befindliche R¹ gemeinsam mit R² eine -CH=CH-CH=CH-Brücke bilden,
durch Umsetzung von Pyrrolen der Formel (II) 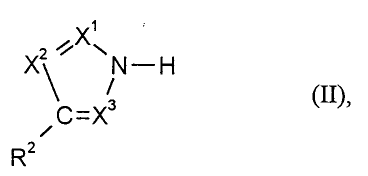 in der die verwendeten Symbole die bei Formel (I) angegebene Bedeutung haben,
mit Phosgen in einem Lösungsmittel, dadurch gekennzeichnet, daß man die Umsetzung bei 50 bis 120°C und in einem aromatischen Lösungsmittel durchführt, das durch Andestillieren entwässert worden ist.

2. Verfahren nach Anspruch 1, dadurch gekennzeichnet, daß man nur ein Azol der Formel (II) einsetzt und so ein Carbonyldiazol der Formel (I) erhält, bei dem die beiden Azolringe identisch sind.

3. Verfahren nach Ansprüchen 1 und 2, dadurch gekennzeichnet, daß man als Azol der Formel (II) Imidazol, Benzimidazol, Pyrazol oder 1,2,4-Triazol einsetzt.

4. Verfahren nach Ansprüchen 1 bis 3, dadurch gekennzeichnet, daß man pro Mol Azol der Formel (II) 0,2 bis 0,3 Mol Phosgen einsetzt.

5. Verfahren nach Ansprüchen 1 bis 4, dadurch gekennzeichnet, daß man als Lösungsmittel Benzol, Toluol, Xylole, Monochlorbenzole, Dichlorbenzole, Trichlorbenzole oder Gemische dieser Lösungsmittel einsetzt.

6. Verfahren nach Ansprüchen 1 bis 5, dadurch gekennzeichnet, daß man das jeweilige Lösungsmittel durch Andestillieren entwässert, indem man so lange auf Siedetemperatur erhitzt, bis kein Austragen von Wasser mehr beobachtet wird.

7. Verfahren nach Ansprüchen 1 bis 6, dadurch gekennzeichnet, daß man bei Reaktionstemperaturen im Bereich 60 bis 100°C arbeitet.

8. Verfahren nach Ansprüchen 1 bis 7, dadurch gekennzeichnet, daß man das nach der Umsetzung vorliegende Reaktionsgemisch aufarbeitet, indem man das gebildete Azolhydrochlorid bei normaler oder erhöhter Temperatur durch Filtration abtrennt und aus der Mutterlauge durch Eindampfen das hergestellte N,N'-Carbonyldiazol in fester Form erhält.

## Claims

1. Process for the preparation of N,N'-carbonyl-diazoles of the formula (I) 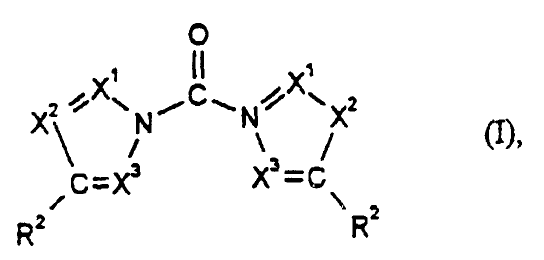 in which
X¹, X² and X³, independently of one another, are each CR¹ or nitrogen, where R¹ is hydrogen or C₁-C₆-alkyl and
R² is hydrogen, or
X¹ and X³ are CR¹,
where the R¹ on X¹ is hydrogen or C₁-C₆-alkyl, and the R¹ on X³ forms an -CH=CH-CH=CH-bridge together with R²,
by reacting pyrroles of the formula (II) 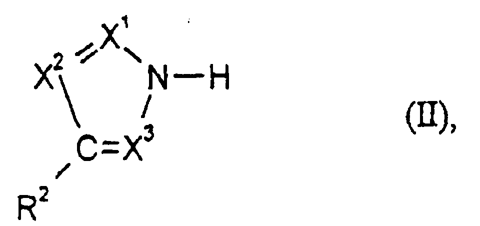 in which the symbols are as defined under the formula (I),
with phosgene in a solvent, characterized in that the reaction is carried out at from 50 to 120°C and in an aromatic solvent which has been dried by incipient distillation.

2. Process according to Claim 1, characterized in that only one azole of the formula (II) is employed, and thus a carbonyl diazole of the formula (I) is obtained in which the two azole rings are identical.

3. Process according to Claims 1 and 2, characterized in that the azole of the formula (II) is imidazole, benzimidazole, pyrazole or 1,2,4-triazole.

4. Process according to Claims 1 to 3, characterized in that from 0.2 to 0.3 mol of phosgene are employed per mol of azole of the formula (II).

5. Process according to Claims 1 to 4, characterized in that the solvent employed is benzene, toluene, xylenes, monochlorobenzenes, dichlorobenzenes, trichlorobenzenes or mixtures of these solvents.

6. Process according to Claims 1 to 5, characterized in that the respective solvent is dried by incipient distillation by heating at the boiling point until no further removal of water is observed.

7. Process according to Claims 1 to 6, characterized in that the reaction temperatures are in the range from 60 to 100°C.

8. Process according to Claims 1 to 7, characterized in that the reaction mixture present after the reaction is worked up by removing the azole hydrochloride formed by filtration at normal or elevated temperature, and obtaining the N,N'-carbonyldiazole prepared in solid form from the mother liquor by evaporation.

## Revendications

1. Procédé pour la préparation de N,N'-carbonyldiazoles de la formule (I) 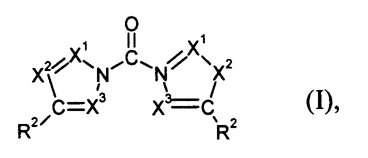 dans laquelle
X¹, X² et X³ représentent indépendamment à chaque fois CR¹ ou un atome d'azote, R¹ étant un atome d'hydrogène ou un groupe alkyle en C₁-C₆ et
R² représente un atome d'hydrogène ou
X¹ et X³ représentent CR¹,
le R¹ se trouvant en X¹ représentant on atome d'hydrogène ou un groupe alkyle en C₁-C₆ et le R¹ se trouvant en X³ formant avec R² un pont -CH=CH-CH=CH-,
par réaction de pyrroles de la formule (II) 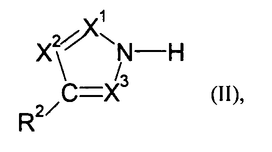 dans laquelle les symboles utilisés ont la signification indiquée pour la formule (I),
avec du phosgène dans un solvant, caractérisé en ce que l'on réalise la réaction à de 50 à 120°C et dans on solvant aromatique, lequel a été déshydraté par distillation.

2. Procédé selon la revendication 1, caractérisé en ce que l'on n'utilise uniquement un azole de la formule (II) et on obtient ainsi un carbonyldiazole de la formule (I), dans lequel les deux cycles azoles sont identiques.

3. Procédé selon les revendications 1 et 2, caractérisé en ce que l'on utilise comme azole de Ia formule (II) l'imidazole, le benzimidazole, le pyrazole ou le 1,2,4-triazole.

4. Procédé selon les revendications 1 à 3, caractérisé en ce que l'on utilise par mole d'azole de la formule (II) de 0,2 à 0,3 mole de phosgène.

5. Procédé selon les revendications 1 à 4, caractérisé en ce que l'on utilise comme solvant do benzène, du toluène, du xylène, du monochlorobenzène, du dichlorobenzène, du trichlorobenzène ou des mélanges de ces solvants.

6. Procédé selon les revendications 1 à 5, caractérisé en ce que l'on déshydrate le solvant correspondant par distillation en le chauffant jusqu'a la température d'ébullition jusqu'a ce que l'on n'observe plus d'évacuation d'eau.

7. Procédé selon les revendications 1 à 6, caractérisé en ce que l'on travaille à des températures de réaction dans le domaine de 60 à 100°C.

8. Procédé selon les revendications 1 à 7, caractérisé en ce que l'on traite le mélange réactionnel présent après la réaction en séparant l'hydrochlorure d'azole formé à température ambiante ou à température élevée par filtration et on obtient le N,N'-carbonyldiazole préparé dans une forme solide par évaporation à partir de la suspension-mère.
